# EUROPEAN PATENT SPECIFICATION

(11) **EP 2 774 933 B1**
(45) Date of publication and mention of the grant of the patent: **21.02.2018**
(21) Application number: 12845071.5
(22) Date of filing: 12.07.2012
(51) Int. Cl.: C07J 41/00, C07J 21/00, C07J 75/00, C07J 71/00

(54) **ULIPRISTAL ACETATE PREPARATION METHOD AND INTERMEDIATE THEREOF**
HERSTELLUNGSVERFAHREN FÜR ULIPRISTALACETAT UND ZWISCHENPRODUKTE DAVON
PROCÉDÉ DE PRÉPARATION D'ACÉTATE D'ULIPRISTAL ET INTERMÉDIAIRE ASSOCIÉ

(30) Priority: 01.11.2011 CN 201110339479
(43) Date of publication of application: 10.09.2014
(73) Proprietor: Utopharm (Shanghai) Co., Ltd, Shanghai 200232 (CN); Changzhou No.4 Pharmaceutical Factory Co., Ltd., Changzhou, Jiangsu 213004 (CN)
(72) Inventor: LUO, Junzhi, Shanghai 200232 (CN); SUN, Yongqiang, Changzhou Jiangsu 213004 (CN); LUO, Xun, Shanghai 200232 (CN); YAN, Yimin, Changzhou Jiangsu 213004 (CN); WANG, Zhaojun, Shanghai 200232 (CN); QIAN, Mingxia, Changzhou Jiangsu 213004 (CN); TU, Yongrui, Changzhou Jiangsu 213004 (CN)
(74) Representative: Török, Ferenc
(86) International application number: PCT/CN2012/000952
(87) International publication number: WO 2013/063859

(56) References cited:
- WO-A1-99/45022
- WO-A2-01/74840
- WO-A2-01/74840
- CN-A- 101 466 723
- CN-A- 102 516 345
- US-A- 5 929 262
- Issei Nitta ET AL: "The syntheses of the corticoid side chain. I. An improved method for the preparation of 17.ALPHA.-hydroxyprogesterone from androst-4-ene-3,17-dione.", Bulletin of the Chemical Society of Japan, vol. 58, no. 3, 1 January 1985 (1985-01-01), pages 978-980, XP055157972, ISSN: 0009-2673, DOI: 10.1246/bcsj.58.978

## Description

### Background of the Present Invention

### Field of Invention

The present invention relates to a medicine, particularly to a method for preparing medicine, and more particularly to a method for preparing ulipristal acetate with an anti-progesterone and anti-glucocorticoid function and to a key intermediate and a preparing method therefor.

### Description of Related Arts

Ulipristal acetate (a compound I; chemical name: 17α-acetoxyl-11β-(4-N,N-dimethylamino-phenyl)-19-norpregna-4,9-diene-3,20-dione) is a strong anti-progesterone and anti-glucocorticoid medicine. A formula of Ulipristal acetate is as follows:

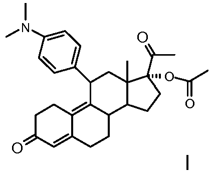

Ulipristal acetate has been approved to be sold in Europe and America for being used within five days after unprotected sex and a known or suspected contraceptive failure, Ulipristal acetate is an effective and safe emergency contraceptive.

Related reports of methods for preparing Ulipristal acetate are as follows.
1. A method disclosed in the United States patent US4954490, (as illustrated in the equation I)
   The method utilizes 3-methoxyl-19-norpregna-1,3,5(10),17(20)-tetraene as the starting material, and after an addition reaction, oxidation, reduction, hydrolysis and an addition-elimination action, 17α-hydroxyl-19-norpregna-4,9-diene-3,20-dione (a compound V2) is obtained by the oxidation, then Ulipristal acetate (the compound I) is obtained by a total of ten reactions comprising ethylene glycol condensation, m-chloroperoxy benzoic acid epoxidation, Grignard addition, acid hydrolysis and acetylation, and a product with a melting point of 118∼121°C is obtained by water/methanol recrystallization. The method is not adaptable to an industrialized production because the steps of the method are too many, the starting material is not easy to be obtained, reaction conditions are complex, some intermediates need to be chromatography purified, its total yield is only 0.62%, costs are very high and the product is not stable enough to be utilized in the medicine; 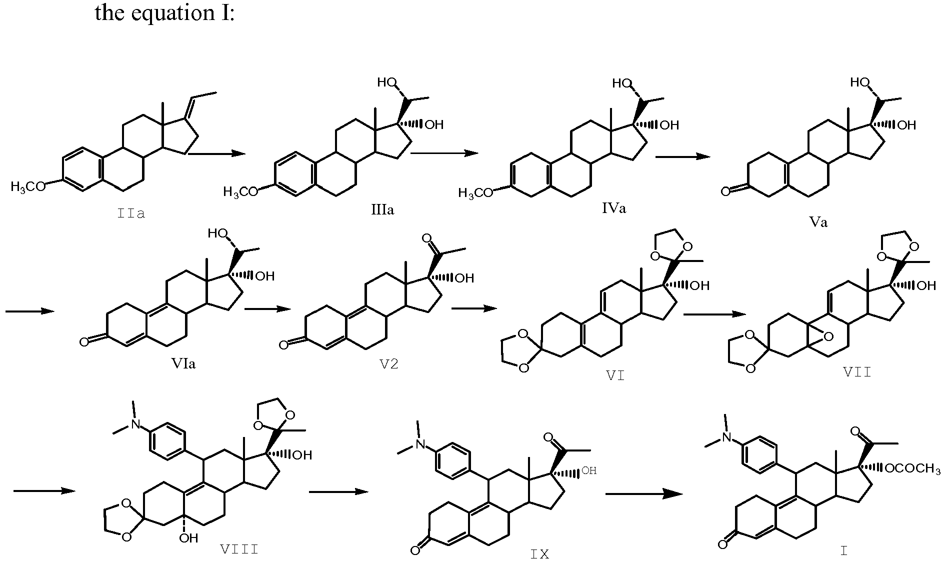
2. Another method disclosed in the United States patent US5929262 (as illustrated in the equation II) 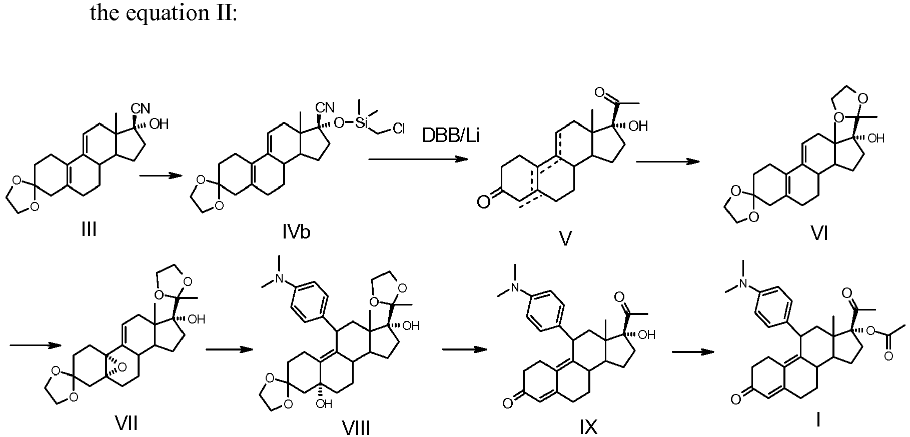 the method utilizes 3,3-ethylendioxyl-17β-cyano-19-norpregna-5(10),9(11)-diene-17α-alcohol (a compound III) as the starting material, and 17α-hydroxy is protected by dimethyl chloromethyl silicane, the starting material is acid hydrolyzed after a reaction with a DBB/Li reagent at a low temperature of -70°C, then diketal is obtained by the ethylene glycol condensation reaction, a desired product is obtained by the epoxidation reaction, the Grignard reaction, the acid hydrolysis reaction and the acetylation reaction, and a yellow product with a melting point of 183∼185°C is obtained by isopropanol, ethyl acetate and ethyl ether crystallization treatments. The method also does not adapt to the industrialized production because a cost of the starting material and DBB are very high, the reaction conditions are strict, an ultra low temperature and anhydrous anaerobic reaction are needed, the yield is low (wherein the total yield is only 14%) and the costs are also very high.
3. The third method was disclosed in the PCT application WO2004078709, (as illustrated in the equation III), a desired product is obtained by utilizing 17α-hydroxyl-19-norpregna-4,9(10)-diene-3,17-dione (a compound V2) with the acetylization, 3-carbonyl condensation, the epoxidation and the hydrolysis. The route of synthesis is simple, but the starting material is prepared from a compound VI by hydrolysis under an acidic condition, the total yield is only 11.8% (calculated from the compound VI), and in fact, its steps are much more, the yield is lower and the costs are higher, therefore, the method is not adaptable to the industrialized production; 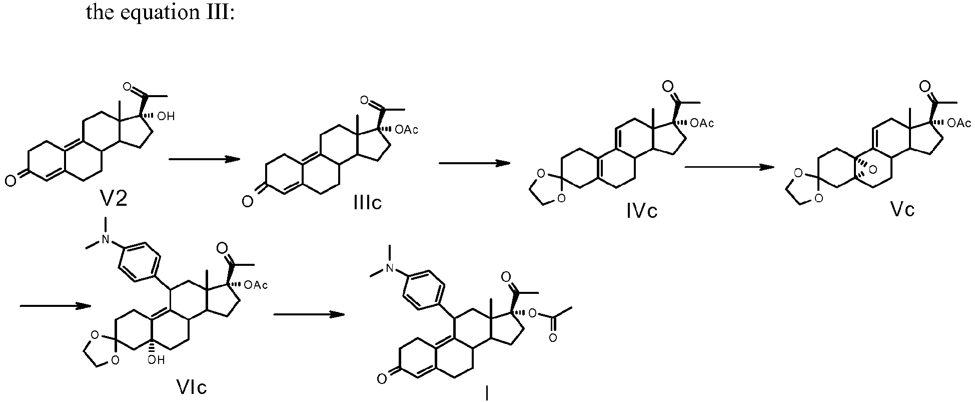
4. A further method was disclosed in the Chinese patent application CN200780021915.9 (as illustrated in the equation IV);

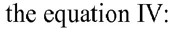

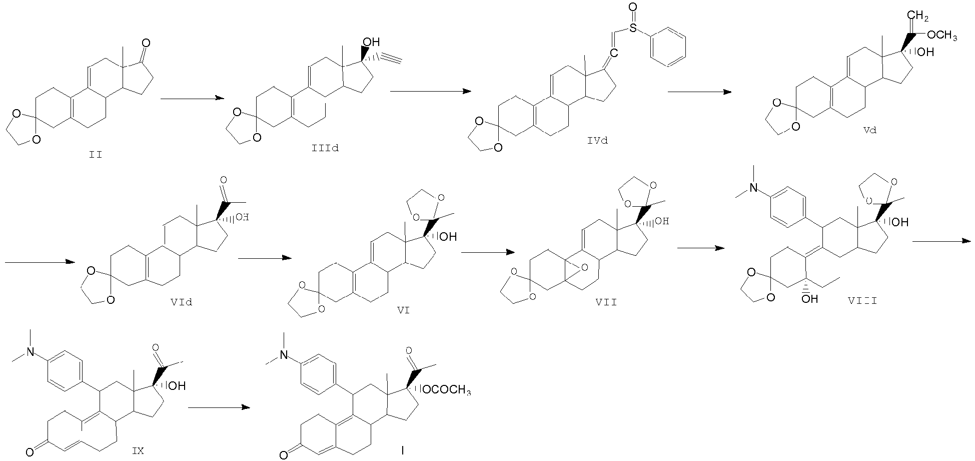

The method utilizes 3,3-ethylendioxyl-19-norpregna-5(10),9(11)-diene-17-one (3-Ethylene Ketal for short, a compound II) as the starting material, the desired product is obtained by a total of nine reactions comprising the acetylene addition reaction, reaction with phenylsulfenyl chloride, the sodium methoxide hydrolysis, the acid hydrolysis, the ethylene glycol condensation, the epoxidation, the Grignard reaction, the acid hydrolysis and the acetylation reaction, the solvate-free crystals are obtained after the isopropanol crystallization and being heated by ethanol and water for 14h at 70°C. The method utilizes acetylene with great danger and the phenylsulfenyl chloride with a stink, wherein the phenylsulfenyl chloride is not stable and not easy to be stored, and the impurities produced by decomposition involved in the reactions will lead to the low yield, additionally, the phenylsulfenyl chloride can deeply pollute environments, and new impurities is produced by being heated for a long time at the high temperature in the crystallization reaction, the total yield of the method is 13.8%∼15.8%, the costs are high, therefore, the method is not adaptable to the industrialized production.

In the conventional methods as above, the method 1, 2 and 4 are related to preparation of the compound VI, and the starting material of the method 3 is hydrolyzed from the compound VI.

WO-A-01/74840 discloses a method for the production of Ulipristal acetate starting from compound V as defined herein.

WO-A-99/45022 discloses a method for the production of the compound of formula V as defined herein via 17α-O-silyl protected intermediate.Summary of the Present Invention

An object of the present invention is to provide an industrialized method without above shortcomings for preparing Ulipristal acetate.

Accordingly, in order to accomplish the above object, the present invention provides a method for preparing Ulipristal acetate, and new key intermediates for preparing Ulipristal acetate.

The method of the present invention utilizes 3-Ethylene Ketal (a compound II) capable of being easily obtained in China as the starting material, 17β-cyano group (a compound III) is obtained by an addition reaction in an solvent of the 3-Ethylene ketal with a cyanation reagent, 17α-hydroxy of the compound III is protected and a compound IV is obtained, a compound V is obtained by acid hydrolyzing the compound IV after the compound IV reacts with methyl lithium or a methyl Grignard reagent, 3,3,20,20-bis(ethylendioxyl)-17α-hydroxyl-19-norpregna-5(10),9(11)-diene (a compound VI) is obtained after the compound V reacting with ethylene glycol in the presence of p-toluenesulfonic acid and trimethyl orthoformate or triethyl orthoformate, then 3,3,20,20-bis(ethylendioxyl)-17α-hydroxyl-5α,10α-epoxy-19-norpregna-9(11)-ene (a compound VII) is obtained by oxidizing the compound VI with hydrogen peroxide, 3,3,20,20-bis(ethylendioxyl)-5α-17α-dihydroxyl-11β-[4-(N,N-dimethylamino)-phenyl-]-19-norpregna-9(11)-ene (a compound VIII) is obtained by Grignard reaction of the compound VII and 4-(N,N-dimethylamino) phenylmagnesium bromide Grignard reagent, 17α-hydroxy-11β-[4-(N,N-dimethylamino)-phenyl-]-19-norpregna-9(11)-diene-3,20-dione (compound IX) is obtained by hydrolyzing the compound VII under an acid condition, Ulipristal acetate (a compound I) is obtained after the compound IX reacts with an acetylation reagent comprising anhydrous acetic acid, perchloric acid and acetic anhydride, reaction steps of the method for preparing Ulipristal acetate (a compound I) is recrystallized with an ethanol: isopropanol (0.5∼1:9), the route of synthesis is as the following: 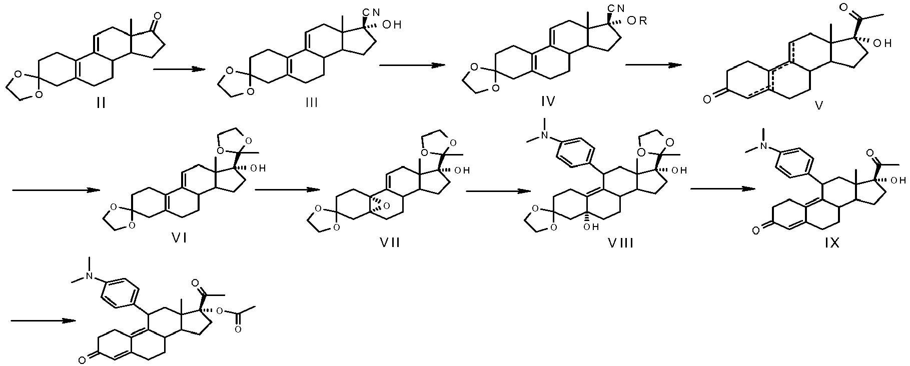 wherein R is a hydroxyl protective group, and is selected from --CH(CH₃)OR₁, or 2-tetrahydropyran, R₁ is selected respectively from C1-C10 alkyl or aryl radical;
dotted lines in a formula V represents that locations of double bonds are at 5(10), 9(11) or 4(5), 9(10);
wherein when the R is -CH(CH₃)OR₁ or 2-tetrahydropyran, compounds of formula IV comprises an isomer compound IV1, IV2 or IV4 or racemic modifications thereof, the detailed formula is as follows: 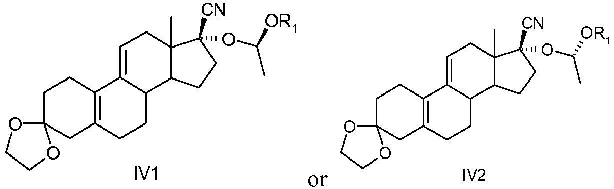 or racemic modifications; or

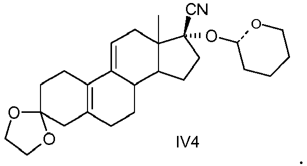

The R₁ is defined as above.

Preferably, the compounds are as follows:

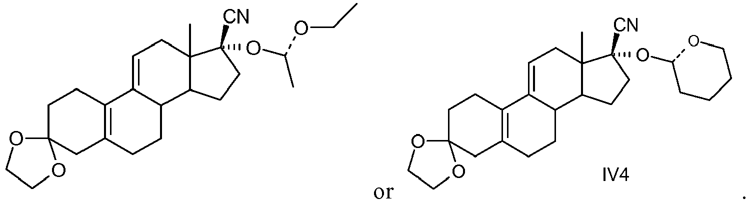

Specifically, the method of the present invention comprises steps of:
a) utilizing 3,3-ethylendioxyl-19-norpregna-5(10),9(11)-diene-17-dione (3-Ethylene Ketal for short) as a raw material and utilizing alcohols as a reacting solvent, wherein a 17β-cyano group compound III is selectively obtained by a reaction of the starting material and a cyanation reagent under a acid condition at a temperature between -10°C to room temperature; wherein the alcohols reaction solvent is selected from methanol, ethanol and isopropanol, and the methanol is preferred; the cyanation reagents are selected from sodium cyanide, potassium cyanide, acetone cyanohydrin and hydrogen cyanide etc, and the sodium cyanide and the potassium cyanide are preferable choices; the acid is preferably selected from formic acid or anhydrous acetic acid; the reacting temperature is preferably selected from -10∼25°C, a mole ratio of the starting material and the cyanation reagents is 1:1.1∼1.5, a reacting time is 2∼24h;
b) obtaining the compound IV by the reaction of the compound III and a hydroxy protective group reagent under the acid condition in a solvent; wherein the solvent is selected from halogenated hydrocarbon such as dichloromethane and chloroform, or ether such as THF (tetrahydrofuran), ethyl ether or isopropyl ether, the solvent is preferably selected from dichloromethane, THF or ethyl ether; the hydroxy protective group reagents are selected from organosilyl matter such as trimethylsilyl lithium, trimethyl chlorosilane and chlorochloromethyl dimethylsilane, or vinyl ether CH2=CHOR₁ such as ethyl vinyl ether, n-propyl vinyl ether, n-butyl vinyl ether and methyl vinyl or 2,3-dihydropyran, etc., the hydroxy protective group reagents are preferably selected from ethyl vinyl ether or 2,3-dihydropyran; the acid is selected from p-toluenesulfonic acid, an amount of the acid is 0.1‰∼5%W/W of an amount of the compound III; the reacting temperature is between - 20 and 50°C, the mole ratio of the starting material and the hydroxy protective group reagent is 1:1.1∼2, the reacting time is 0.5∼24h;
c) The 5(10),9(11)-diene-3,20-dione compound V1 or 4,9(10)-dienen-3,20-dione compound V2 or a mixture V comprising the compound V1 and compound V2 was obtained by hydrolyzing the compound IV under the acid condition after reacting the compound IV with a methylation reagent, wherein the reacting solvent is selected from ether such as diethyl ether, isopropyl ether or THF; or halogenerated hydrocarbon of dichloromethane or chloroform, the reacting solvent is preferably selected from diethyl ether, THF or dichloromethane; the methylation reagent is selected from methyl lithium or methyl Grignard reagent, and is preferably selected from methyl lithium; the reacting temperature is -30°C to a reflux temperature and preferably the room temperature; the mole ratio of the starting material and the methylation reagent is 1:1.1∼5, and preferably 2∼3 equivalents; the reacting time is 0.5∼24h; the solvent for hydrolyzing under the acid condition is selected from acetone or butanone; methanol or ethanol; diethyl ether, THF, ethylene glycol or dimethyl ether; acetic ether or menthyl acetate; or dichloromethane or chloroform; the reagent is preferably selected from butanone, methanol, THF or diethyl ether,
   wherein icy water is added for quenching after the reaction, then the acid is directly added for hydrolyzing, or an extracting reagent insoluble in water is added for extracting, wherein the extracting reagent is selected from the halogenerated hydrocarbon such as the dichloromethane or the chloroform, or an esters solvent such as the acetic ether or the menthyl acetate, or the ether such as the diethyl ether or the isopropyl ether, or an arene reagent such as benzene or methylbenzene; the acid is directly added for hydrolyzing after extraction, or a reagent soluble in water and the acid are added for hydrolyzing after concentrating, wherein the acid utilized in hydrolyzing is selected from mineral acids such as sulphuric acid, hydrochloric acid, potassium bisulfate or sodium bisulfate, or organic acids such as formic acid or acetic acid, 1∼6N hydrochloric acid is a preferable choice; a hydrolyzing temperature is -40∼100°C, preferably 25∼50°C,
   the invention further discloses that by controlling the hydrolyzing temperature, a main product of the hydrolysis is the compound V1, and then the compound V1 is slowly transformed to the more stable compound V2, or the pure compound V2 can be obtained by crystallizing with the solvent such as methanol,etc; in fact, the ratio of the compound V1 and V2 can be modified freely by controlling the hydrolysis conditions such as acidity, the temperature and the reacting time, but the ratio has no effect on the next step; taking the ethyl vinyl ether as the protective group is an example, the compound of formula IV is hydrolyzed and transformed into imine after the addition reaction with the methyl lithium or methyl Grignard reagent, the compound V (the compound V is the compound V1, the compound V2 or a mixture of the compound V1 and V2) is obtained after the imine is further hydrolyzed, 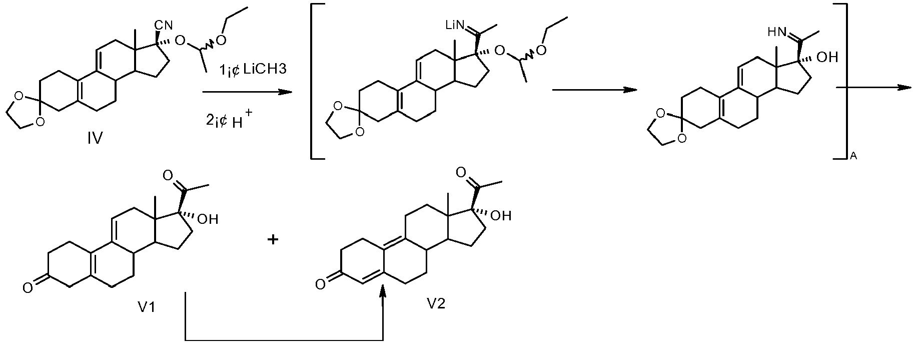 additionally, in the procedure of the step c), the compound IV can be involved in a next step reaction as a single isomer or a mixture of isomers, preferably the mixture of isomers;
d) the 3,20-diketal compound VI is obtained from compound V in ketal formation reacting with ethylene glycol on the presence of p-toluenesulfonic acid and trimethyl orthoformate or triethyl orthoformate at the room temperature, wherein the solvent is preferably selected from dichloromethane, the reacting temperature is 0°C to the room temperature, the reacting time is 1∼8h;
e) the compound VII is obtained by reacting comound VI with oxidant in dichloromethane under an alkaline condition and perhalogeno-acetone at the room temperature, , wherein an alkali is selected from pyridine, dipotassium phosphate, monopotassium phosphate, diposodium phosphate, monosodium phosphate, etc.; the oxidant is selected from hydrogen peroxide, m-chloroperoxybenzoic acid, etc., preferably hydrogen peroxide; the reacting temperature is -10∼10°C;
f) the compound VIII is synthesized from compound VII and a 4-(N,N-dimethylamino)phenylmagnesium bromide Grignard reagent via Grignard reaction in the presence of cuprous chloride catalyst, wherein the mole ratio of the starting material and the Grignard reagent is 1:1.5∼5, the reacting temperature is -10∼40°C, the reacting time is 2∼8h;
g) the obtained compound VIII is hydrolyzed with dilute acid with in dichloromethane at a temperature of 0-25°C , wherein the acid is selected from the mineral acids such as hydrochloric acid, sulphuric acid or sodium bisulfate, preferably 0.2∼4N HCl solvent, the reacting temperature is -10∼50°C, the reacting time is 1∼5h;
h) acetylating the compound IX with the anhydrous acetic acid, perchloric acid or acetic anhydride, preferably the perchloric acid and the acetic anhydride mixed with acetic acid for obtaining Ulipristal acetate (compound I); wherein the reacting temperature is -40∼25°C, preferably -10∼25°C; the ratio of the acetic acid is 1∼50%V/V, preferably 10-15%V/V, of the acetylation reagent comprising acetic acid, perchloric acid and acetic anhydride, the reaction can be presented at 0∼25°C without side reactions once acetic acid is added.However, the similar reaction in the reference CN200780021915.9 need to be presented at -30∼-20°C;
i) crystallizing the crude Ulipristal acetate with the ethanol and the isopropanol for obtaining Ulipristal acetate with a purity of over 99%;
   wherein in the step b), a method for preparing a key intermediate Ulipristal acetate of formula IV is provided;
   wherein in the step c), a method for preparing another key intermediate compound of Ulipristal acetate of formula V is provided.

A preferred embodiment of the present invention for preparing the key intermediate compound of formula V is provided, wherein the compound III reacts with the hydroxy protective group reagent for obtaining the compound of formula IV, the alkali is directly added for modifying a pH value to 7∼8 without separation, then the compound IV reacts with the methyl lithium or the methyl Grignard reagent, the product is hydrolyzed in the solvent under the acid condition right after the reaction or after being processed for obtaining the compound V; wherein the hydroxy protective group reagent is selected from the acid anhydride, the acid or the acyl chloride, vinyl ether such as ethyl vinyl ether, n-propyl vinyl ether, n-butyl vinyl ether, isobutyl vinyl ether and methyl vinyl ether, or 2,3-dihydropyran, etc.

Another preferred embodiment of the present invention for preparing Ulipristal acetate is provided, wherein the compound VI continuously reacts without separating the various intermediates for obtaining Ulipristal acetate, the target product is obtained by one-pot reaction from the step f) to the step i) with a high yield. Detailed steps are concentrating epoxide to a proper volume after the epoxidation reaction, modifying the pH value to 1∼2 after reacting with the Grignard reagent and being quenched by an aqueous solution of ammonium chloride, stirring for 1∼2h for hydrolyzing, then extracting by dichloromethane, washing, being dried by anhydrous magnesium sulfate, being acetylated by the acetylation reagent right after filtering for obtaining the target product, Ulipristal acetate compound I.

The invention further discloses that the intermediate of formula IV is conductive to reacting with methyl lithium or methyl Grignard reagent, the reaction condition is mild, the protective group is easy to be removed after the reaction, the side reactions are few, a post treatment is simple, the yield is high, the reagents are cheap and costs are low. For example, when the hydroxyl protective group reagent of the 17α-hydroxy is selected from ethyl vinyl ether or 2,3-dihydropyran, the compound V is obtained by the reaction of the compound III and the intermediate of formula IV, the yield of a two-step reaction is 70∼75%, the purity is above 98%. Obviously, the intermediate of formula IV is the key intermediate for preparing Ulipristal acetate, and is an important part of the present invention.

The yield of the 3,20-dione (the compound VI) increases by utilizing the key intermediate compound of formula IV for preparing Ulipristal acetate of the present invention, the yield of the compound II to the compound VI is 68%, the costs are low.

Obviously, the procedure of the present invention is simple, there are totally eight steps, the reaction condition is mild and is conductive to reacting, the total yield is about 25∼27%. In fact, the compound IV doesn't need to be separated and Ulipristal acetate could be prepared with high yield by one-pot reaction from the step f) to the step i). As a result, only the isolation of three intermediates of the compound III, V and VI are necessary. The operation is simple and is adaptable to the industrialized production.

The present invention also provided a method for purifying Ulipristal acetate, wherein the method comprises steps of: adding a hot solvent of ethanol: isopropanol (0.5∼1:9) into the crude ulipristal acetate, wherein the solvent is 5∼20 times of the crude Ulipristal acetate; cooling the hot solvent to 0∼25°C for crystallizing, wherein the product has the purity of 99%.

Therefore, our process is simple and itsreaction condition is mild with high yield. The purity of obtained product is high. Moreover, the reagents in our process are cheap and easy to be obtained. As a result, our costs are low., Our process is adaptable to the industrialized production and has a high value in an industrial application.

These and other objectives, features, and advantages of the present invention will become apparent from the following detailed description, the accompanying drawings, and the appended claims.

### Detailed Description of the Preferred Embodiment

The present invention is further illustrated, but not limited, by following preferred embodiments, wherein:
HNMR is Varian INOVA-400 nuclear magnetic resonance spectrometer testing,
Monocrystal testing utilizes a Bruker SMART APEX-II monocrystal X ray diffractometer; testing requirements is: a CuKa radiation, a graphite monochromator, a diameter of a single vessel φ=0.50mm, a detector distance between a crystal and a CCD detector d=60.3mm, a 40kV tube voltage, a 30mA tube current; a scan mode is: Φ/w scanning.

### Example 1, preparing 3,3-(ethylene-dioxy)-17β-cyano-17α-hydroxy-19-norpregna-5(10),9(11)-diene (a compound III):

adding 3-Ethylene ketal (2.0kg, 6.37mol), methanol (12L), sodium cyanide (343g, 7.0mol) and acetic acid (440ml) in a reaction bulb, then stirring overnight at a room temperature, icy water was added and the raction mixture was ftirred for 30min, the precipitated crystalline product was filtered off, washed the filter cake three times with water, then drying for obtaining 2.06kg white powder, mp: 176∼178°C (decomposed), a yield is 95%, HPLC purity is above 98%;
MS: 342(M+1);
an absolute configuration of the compound III is illustrated according to the monocrystal testing:

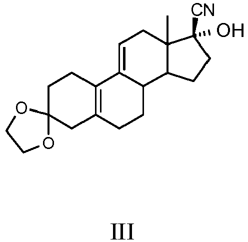

### Example 2, preparing 17α-[(±)1-(1-ethoxyl) ethyl] oxyl-17β-cyano-3,3-(ethylene-dioxy)-19-norpregna-5(10),9(11)-diene:

To a suspension of the compound III (2.0kg, 5.87mol) obtained from Example 1, in THF (14L) and p-toluenesulfonic acid (12.0g, 70mmol), methyl vinyl ether (668ml, 7.04mol) is added at the room temperature by cooling in the icy water. The reaction mixture was stirred for 4h at the same temperature, triethylamine (15ml) and water were added and stirred for 10min, an aqueous phase was extracted by dichloromethane, organic phase was combined and washed with water, dried by anhydrous magnesium sulfate, and concentrated in vacuo for obtaining 2.43kg off-yellow or colourless oil, wherein the yield is quantified (the yield is 100%);
wherein the oil is displayed as two compounds by a TLC (ethyl acetate: petroleum ether=1:5), a solid is precipitated by freezing the oil, products crystallized from ethyl acetate: Petroleum ether (1:2) is mainly high polarity products, HPLC>90%; separating a small amount of the products by a chromatography (with 300∼400 mesh numbers) for obtaining a compound IV1 (with a low polarity) and a compound IV2 (with a high polarity);
wherein the compound IV1 is the off-yellow oil: MS: 315(M+1), 651 (2M+Na); ¹HNMR (CDCl₃): 0.96 (d,3H)), 1.20 (t,3H), 1.24-1.34 (m), 1.37 (d,3H) 1.44-1.50 (m,1H), 1.52 (s,1H), 1.75-1.97 (m,8H), 2.14-2.22 (m), 2.28 (s,2H), 2.50-2.70 (m), 3.46-3.60 (m), 4.0 (s,4H), 5.10 (q,1H), 5.60 (d, 1H);
wherein the compound IV2 is a white crystal: the melting point: 131∼134°C; MS: 315 (M+1), 651 (2M+Na); ¹HNMR (CDCl₃): 0.97 (d,3H)), 1.26 (t,3H), 1.33 (d,3H), 1.45-1.49 (m), 1.52 (s,1H), 1.77-1.97 (m,8H), 2.13-2.28 (m,6H), 2.52 (d,1H), 2.77 (d,1H), 3.55-3.74 (m,2H), 3.98 (s,4H), 5.02 (q,1H), 5.59 (d, 1H).

### Example 3, preparing the 17α-[(±)1-(1-ethoxyl) ethyl] oxyl-17β-cyano-3,3-(ethylene-dioxy)-19-norpregna-5(10),9(11)-diene:

To a suspension of the compound III (50.0g, 0.147mol) obtained from Example 1 in dichloromethane (500ml) and p-toluenesulfonic acid (0.3g, 1.74mmol), methyl vinyl ether (17ml, 0.18mol) is added at the room temperature by cooling in the icy water. The reaction mixture was stirred for 4hwherein 60.7g the off-yellow oil, the 17α-[(±)1-(1-ethoxyl) ethyl]-17β-cyano-3,3-ethylendioxyl-19-norpregna-5(10),9(11)-diene, is obtained by the same operations in Example 2, the yield is quantified, the oil is displayed as the two compounds by TLC, a result of constitution identifying is the same as the result in the preferred embodiment 2.

### Example 4, preparing 17α-[(±)1-(1-n-propyl oxyl) ethyl] oxyl-17β-cyano-3,3-(ethylene-dioxy)-19-norpregna-5(10),9(11)-diene:

utilizing n-propyl vinyl ether as a raw material, wherein the off-yellower oil is obtained by treatments the same as Example 3, the yield is quantified; the oil is displayed as two compounds by the TLC;
MS: 324 (M-OCH (OC₃H₇) CH₃), 368 (M-OC₃H₇), 450 (M+Na), 877 (2M+Na).

### Example 5, preparing 17α-[(±)1-(1-n-butyl oxyl) ethyl] oxyl-17β-cyano-3,3-(ethylene-dioxy)-19-norpregna-5(10),9(11)-diene:

utilizing n-butyl vinyl ether as the starting material, wherein the off-yellower oil is obtained by the treatments the same as Example 3, the yield is quantified; the oil is displayed as two compounds by the TLC;
MS: 324 (M-OCH (OC₄H₉) CH₃), 368 (M-OC₄H₉), 464 (M+Na), 905 (2M+Na).

### Example 6, preparing 17α-[(±)1-(1-isobutyloxyl)ethyl] oxyl-17β-cyano-3,3-(ethylene-dioxy)-19-norpregna-5(10),9(11)-diene:

utilizing isobutyl vinyl ether as the starting material, wherein the off-yellower oil is obtained by the treatments the same as Example 3, the yield is quantified; the oil is displayed as two compounds by the TLC;
MS: 324 (M-OCH (OC₄H₉) CH₃), 368 (M-OC₄H₉), 464 (M+Na), 905 (2M+Na).

### Example 7, preparing 17α-[(±)1-(1-tetrahydropyran) ethyl] oxyl-17β-cyano-3,3-(ethylene-dioxy)-19-norpregna-5(10),9(11)-diene:

utilizing dihydropyran as the starting material, wherein the off-yellower oil is obtained by the treatments the same as Example 3, the yield is quantified; the oil is displayed as two compounds by the TLC;
MS: 426 (M+1), 873 (2M+Na).

### Example 8, preparing a compound V:

adding 17α-[(±)1-(1-ethoxyl) ethyl] oxyl-17β-cyano-3,3-(ethylene-dioxy)-5(10), 9(11)-die (12.0g, 29mmol), anhydrous THF (120ml) in the reaction bulb and cooling in the icy water, 1.0M methyl lithium 2-methyl tetrahydrofuran (58ml, 58mmol) was added at 0∼10°C then stirred at 0∼10°C for 4hr, - 50ml water was added and stirred for 10min, separating out the organic phase and extracting the aqueous phase byethyl acetate , combined the organic phase concentrated in vacuo , then 50ml methanol and 2N HCl were added and stirred for 2h at 25°C. The reaction mixture was poured into the icy water. the organic was separated out, extracting the aqueous phase by ethyl acetate, combining the organic phase, drying, filtering, and concentrating in vacuo for obtaining 6.6g off-yellow powder, mp: 184∼188°C, the yield is 73%, the powder is displayed as two compounds by the TLC (ethyl acetate: petroleum ether=1:2);
wherein ethyl acetate: petroleum ether=1:5 is utilized as an eluant, products are separated by the chromatography for obtaining a compound V1 (with a low polarity) and a compound V2 (with a high polarity), a sample for analyzing is crystallized by ethyl acetate, wherein ethyl acetate is 5 times of the products;
wherein the compound V1 is the off-yellow powder, the MP: 196∼200°C;
MS: 15(M+1); ¹HNMR (CDCl₃): 0.71 (s,3H), 1.28-1.48 (m,2H), 1.64 (m,1H), 1.75-1.81 (dd,1H), 1.89-2.02 (m,5H), 2.22-2.24 (br,1H), 2.27 (s,3H), 2.45-2.53 (m,3H), 2.67-2.80 (m,3H), 2.80 (s,1H), 2.86 (brs,2H), 5.62 (d, 1H);
the absolute configuration of the compound V1 is illustrated according to the monocrystal testing: 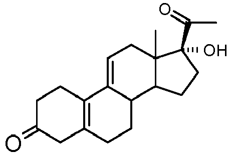 wherein the compound V2 is the off-yellow powder, the MP: 197∼202°C;
MS: 315(M+1); ¹HNMR (CDCl₃): 0.87 (s,3H), 1.34-1.46 (m,2H), 1.48-1.53 (m,1H), 1.59-1.66 (m,1H), 1.83-1.96 (m,4H), 2.10 (dt,1H), 2.26-2.30 (s,3H,m,1H), 2.2-2.56 (m,5H), 2.73 (dt,1H), 2.81-2.91 (m,3H), 5.67 (s, 1H);
the absolute configuration of the compound V2 is illustrated according to the monocrystal testing:

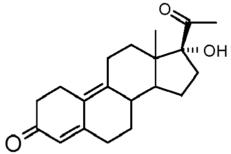

### Example 9, preparing the compound V:

adding the 17α-[(±)1-(1-ethoxyl)ethyl]oxyl-17β-cyano-3,3-ethylendioxyl-5(10),9(11)-dien (10.0g, 24.2mmol), anhydrous THF (100ml) in the reaction bulb and cooling in the icy water, 1.0M methyl lithium 2-methyl tetrahydrofuran (48.4ml, 48.4mmol) was added at 0∼10°C, then stirred for 4h. 10ml the 4N HCl was added and stirred for 30min, The organic phase was separated out and the aqueous phase was extracted by ethyl acetate, combining the organic phase, drying,concentrating in vacuo.and crystallizing from ethyl acetate: petroleum ether to obtain 5.4g the off-yellow powder,mp: 185∼188°C, the yield is 71%;
wherein utilizing 1.6M methyl lithium ethyl ether for reacting, the similar result can be obtained.

### Example 10, preparing the compound V:

utilizing the same method as Example 9, wherein utilizing anhydrous ethyl ether and 1.6M methyl lithium ethyl ether for reacting, the similar result can be obtained, the yield is 74%.

### Example 11, preparing the compound V:

utilizing the 17α-[(±)1-(1-ethoxyl) ethyl] oxyl-17β-cyano-3,3-ethylendioxyl-5(10),9(11)-dien (4.0g, 9.68mmol) as the starting material, stirring for 2h with the temperature kept after replacing the methyl lithium with a new-prepared methyl Grignard reagent, wherein 2.1g the off-yellower powder is obtained by the treatments the same as Example 9, the yield is 68%.

### Example 12, preparing the compound V:

utilizing the 17α-[(±)1-(1-n-propyl oxyl) ethyl] oxyl-17β-cyano-3,3-ethylendioxyl-19-norpregna-5(10),9(11)-diene (8.0g, 18.7mmol) as the starting material, wherein 4.0g the off-yellower powder is obtained by the treatments the same as Example 9, the yield is 70%.

### Example 13, preparing the compound V:

utilizing the 117α-[(±)1-(1-isobutyl oxyl) ethyl] oxyl-17β-cyano-3,3-ethylendioxyl-19-norpregna-5(10),9(11)-diene (10.0g, 22.7mmol) as the starting material, wherein 4.7g the off-yellower powder is obtained by the treatments the same as Example 9, the yield is 67%.

### Example 14, preparing the compound V:

utilizing the compound (21.3g, 50.1mmol) prepared in the preferred embodiment 7 as the starting material, wherein 11.2g the off-yellower powder is obtained by the treatments the same as Example 9, the yield is 72%.

### Example 15, preparing the compound V2:

Adding 1.6M methyl lithium ethyl ether (380ml, 0.61mol) into the 17α-[(±)1-(1-ethoxyl)ethyl]oxyl-17β-cyano-3,3-(ethylene-dioxy)-5(10),9(11)-dienne (50.0g, 0.121mmol) in ethyl ether (500ml) at 0∼10°C, then the reaction mixture was stirred for 4h and poured into the ice water, the organic phase was separated out, extracting the aqueous phase by ethyl acetate until no product is generated, the organic phase was combined ,washed once with water and dried, then concentrated in vacuoto obtain 58g of product, 20-fold acetone and 80ml 4N HCl was added, and stirred for 8h at 25°C, then the reaction mixture was poured into 5 times ice water.The aqueous phasewas extracted by dichloromethanethe organic phase was combined and washed once by saturated sodium bicarbonate and once by water, , dried and concentrated in vacuo 50ml ethyl acetate was addied and cooled, then stirred for 30min and the precipitated crystalline was filtered off, to obtain27.4g off-yellow solids, mp: 195∼199°C, the yield is 72%; the solid is displayed as the compound V2 by the TLC, the HPLC is 98%.

### Example 16, preparing the compound V:

To a solution of the compound III (1.5kg, 4.40mol) in THF (7.5L), p-toluenesulfonic acid (5g) and ethyl vinyl ether (632ml, 6.6mol) were added at the room temperature by cooling in the icy water., the reaction mixture was stirred for 3h at the room temperature, then cooled to 0°C and modifying PH to neutral with the triethylamine, 1.6M methyl lithium ethyl ether (8.0L, 12.8mol) was added at 0∼10°C. the reaction mixture was stirred for 8h at the same temperature. 300ml 2N HCl was added slowly and stirred for 4h at 25°C, the organic phase was separated. the aqueous phase was extracted six times by acetic ether, 600ml *6; the organic phase was combined, washed with water and dried, then concentrated in vacuo to the constant weight, 5 times of acetic ether was added and dissolved by heating, then cooled, the precipitated crystalline was filtered off to obtain1036g of the off-yellow powder, mp: 183∼187°C, the yield is 75%, the HPLC shows that about 15% of the product is V1 and about 83% of the product is V2.

### Example 17, preparing 3,3,20,20-bis(ethylene-dioxy)-17α-hydroxy-19-norpregna-5(10),9(11)-diene (a compound VI):

To the solution of the compound V (1035g, 3.30mol) in dichloromethane (11L), ethylene glycol (1000ml, 17.9mol), trimethyl orthoformate (1400ml, 8.4mol) and p-toluenesulfonic acid (30g, 0.15mol)were added, at 25°C, wherein the starting material disappeared after reacting for 5h at the room temperature, the reaction mixture was poured into the saturated sodium bicarbonate (5kg) and stirred for 30min, wherein the aqueous phase was extracted twice 2L *2, the organic phase was combined and washed with water, then dryied with the anhydrous magnesium sulfate, 10ml pyridine was added, it was concentrated in vacuo at 40°C until the dichloromethane disappears, 600ml the methanol was added,, then cooled to 0∼10°C and stirred for 30min, the precipitated crystalline was filtered off and dried to obtain 1192g of the compound wherein the yield is 90%.

### A preferred embodiment 18, preparing 3,3,20,20-bis(ethylene-dioxy)-17α-hydroxy-5α,10α-epoxy-19-norpregna-9(11)-ene (a compound VII):

To the solution of the compound VI (1190g, 2.96mol) in dichloromethane(12L), the pyridine (20ml) and hexafluoroacetone trihydrate (270ml, 1.93mol)were added, the reaction mixture was cooled to 0∼5°C, 50% H₂O₂(970ml, 20mol) was added slowly, keeping the temperature at -5∼5°C, stirred for 3∼4h until the starting material disappeared, the organic phase was separated , the aqueous phase was extracted twice with the dichloromethane, the organic phase was combined and washed once by 10% sodium thiosulfate aqueous solution (500ml), then washed with water(500ml*2),dried the organic phase with the anhydrous magnesium sulfate, then the organic phase was concentrated in vacuo until the constant weight of 1310g (the constant weight is 1237g according to a theory) is reached, providing the product, 5α,10α-epoxy:5β,10β-epoxy=8:2, (the product is detected by the HPLC), which was used in the next step without purification.

### Example 19, preparing 3,3,20,20-bis(ethylene-dioxy)-5α-17α-dihydroxy-11β-[4-(N,N-dimethylamino)-phenyl-]-19-norpregna-9(11)-ene (a compound VIII):

To a mixture of Mg turnings(165g, 6.87mol) in 1,2-dichloromethane (2ml) and THF (200ml), 4-bromine-N,N-dimethylaniline (1380g, 6.9mol) and THF (3000ml) was dropped slowly at such a rate to keep the temperature between 40-50°C, then the mixture was stirred at 40-50°C for 3h to obtain the gray Grignard reagent, which was cooled to 25°C, cuprous chloride (43g, 0.44mol) was added by cooling with icy water, the solution of epoxy (the epoxy comprises about 2.3mol the 5α,10α-epoxy) prepared in Example 20 in dichloromethane(4L) was dropped slowly and kept the temperature between 10 and 20 °C, then stirred for 2h, the reaction mixture was poured into a 3000ml icy saturated NH₄Cl and stirred for 10mins, the organic phase was separated out, wherein the aqueous phase is extracted by dichloromethane, 2000ml*5. The organic phase was combined and washed three times with water, then dried with the anhydrous magnesium sulfate, the organic phase was concentrated in vacuo until the organic phase was in a foam, 800ml of ethyl acetate was added the and heated at 70°C for 10mins, then cooled to 10α20°C and stirred for 30mins, the precipitated crystalline was filtered off, washed twice with ethyl acetate and dried to obtain 957g of off-white powder, wherein the yield of a two-step reaction is 60%, mp: 230∼234°C, HPLC>95%.

### Example 20, preparing 17α-hydroxy-11β-[4-(N,N-dimethylamino)-phenyl-]-19-norpregna-4,9(10)-diene-3,20-dione (compound IX):

To the solution of 2N HCl (4000ml), the compound VIII (950g, 1.76mol) prepared in Example 21 was added, the mixture was stirred at 25°C for 2h until the starting material disappeared(by TLC), the reaction mixture was extracted five times with the dichloromethane 3000ml*2, 1000ml*3, the starting material the organic phase was combined and washed once with the saturated sodium bicarbonate and once with the water, then dried with the anhydrous magnesium sulfate, filtered and concentrated to about 3000ml for a further usage.

### Example 21, preparing Ulipristal acetate (I):

To a solution of compound IX prepared in Example 22 in dichloromethane, acetic acid (200ml, 3.50mol) was added. The mixture was cooled to -10°C, then 70% perchloric acid(237ml, 3.92mol) was added, , acetic anhydride (1400ml, 14.9mol) was dropped slowly at 0∼10°C while stirring, stirred continue for 1∼2h, 3kg of icy water was added, the organic phase was separated, the aqueous phase was extracted six times with dichloromethane, 500ml*6., The combined organic phase was washed once with 800ml saturated sodium bicarbonate and once with 800ml the water, then dried with anhydrous magnesium sulfate, filtered off, and the filtrate was concentrated in vacuo until the constant weight is reached, 800ml isopropanol was added and stirred for 30min, the precipitated crystalline was filtered off and washed with isopropanol, then dried at 60°C to obtain 790g of the off-yellow solids, which was dissolved in 8000ml of isopropanol: ethanol (95:5) by heating, decolourized with 1% activated carbon, the mixture was filtered and cooled to 10°C and stirred for 1h, the precipitated crystalline was filtered off and washed with isopropanol: ethanol (95:5), and dried at 60°C to obtain586g the off-yellow solids, mp: 151∼153°C, Ulipristal acetate was confirmed by structure, the yield is about 70%, the HPLC>99%.

### Example 22, preparing Ulipristal acetate (I):

To a solution of 3,20-diketal (100g, 0.25mol) compound VI in dichloromethane (1L), pyridine (5ml) and hexafluoroacetone trihydrate (20ml, 143mmol)was added and cooled to -10∼0°C, 50% H₂O₂ (70ml, 1.44mol) was added slowly, the mixture was stirred at -5∼5°C for 3∼4h until the starting material disappeared, the organic phase was separated out, the aqueous phase was extracted twice with dichloromethane, the combined organic phase was washed with 10% sodium thiosulfate (10ml), the organic phase was washed with water(50ml*2), then dried with anhydrous magnesium sulfate, and flitered, the filtrate was concentrated in vacuo to a volume of 200ml and the so obtained solution was used in the next step.

To the mixture of Mg turnings(9.6g, 0.4mol) in 1,2-dichloromethane (1ml) and THF (50ml),, the solution of 4-bromine-N,N-dimethylaniline (71g, 0.35mol) and THF (200ml) was dropped slowly at such a rate to keep the temperature between 40-50°C, then the mixture was stirred at 40-50°C for 3h to obtain the gray Grignard reagent, which was cooled to 25°C, cuprous chloride (3g, 30mol)was added, the mixture was cooled to 10∼20°C by icy water, the solution of epoxide in dichloromethane was dropped slowly to keep below20°C, and stirring for 2h at the same temperature, the reaction mixture was poured into 500ml of icy saturated NH₄Cl and stirred for 10mins, the organic phase was separated out, wherein the aqueous phase was extracted by dichloromethane 5 times(400ml*5), the combined organic phase was washed with1000ml saturated NHCl solution, then stirred for 2h at 25°C to separat out the organic phase, wherein the aqueous phase was extracted by the dichloromethane 3 times(200ml*3), the organic phase was combined, then washed with the saturated sodium bicarbonate and once with the water, dried with anhydrous magnesium sulfate, filtered, the filtrate was poured into the reaction kettle, anhydrous acetic acid and 70% the perchloric acid (24ml, 295mol) (18ml, 315mmol) were added and cooled to-10∼0°C by icy water, , acetic anhydride (140ml, 1.49mol) was dropped slowly to keep temperature at -10∼0°C, stirred continue for 30min at the same temperature , wherein the aqueous phase was extracted by dichloromethane 3 times(200ml*3), the combined organic phasewas washed with the saturated sodium bicarbonate and once with the water, then dried the organic phase with anhydrous magnesium sulfate, filtered, the filtrate was concentrated to the constant weight of 110g, which was recrystallized with the 10-fold ethanol: isopropanol (95:5) reagent to obtain 61.2g the off-yellow crystal, mp:145∼148°C ; the yield is 52%, the HPLC>97%; recrystallized again with the ethanol: isopropanol (95:5) reagent and dried at 60°C to obtain 46.0g the off-yellow crystal, wherein Ulipristal acetate was confirmed by structural testing, the yield is 75%, HPLC>99%.

One skilled in the art will understand that the embodiment of the present invention as shown in the drawings and described above is exemplary only and not intended to be limiting.

## Claims

1. A method for preparing Ulipristal acetate, comprising reaction steps of: 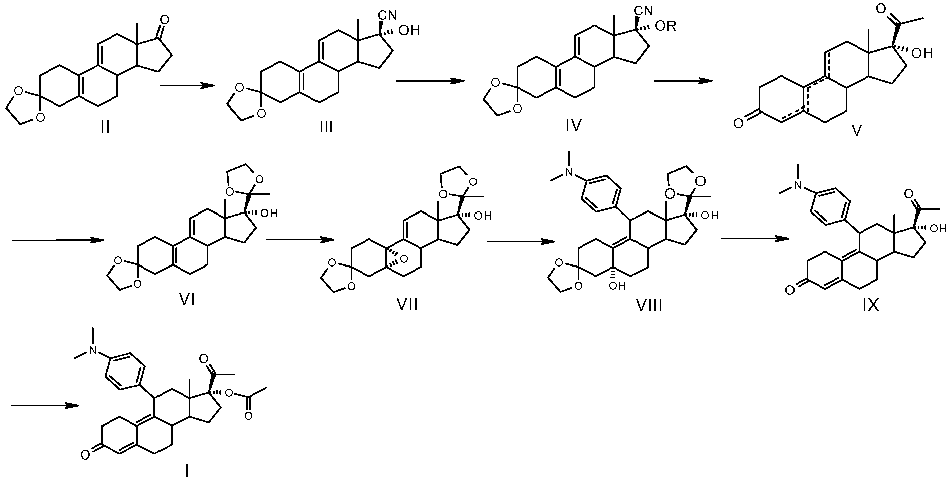 wherein R is a hydroxyl protective group, and is selected from -CH(CH₃)OR₁, or 2-tetrahydropyran, R₁ is selected respectively from C1-C10 alkyl or aryl radical; dotted lines in formula V represents that locations of double bonds are at 5(10), 9(11), or 4(5) ,9(10), wherein the method comprises steps of:
a) utilizing 3,3-(ethylendioxyl)-19-norpregna-5(10),9(11)-diene-3,17-dione of formula II as a raw material and utilizing alcohols as a reacting solvent, wherein 3,3-(ethylendioxy)-17β-cyano-7α-hydroxyl-19-norpregna-5(10),9(11)-diene of formula III is selectively obtained by a reaction of the starting material and a cyanation reagent under a weak acidic condition at a temperature between -10°C to room temperature;
b) obtaining the compound of formula IV by the reaction of the compound of formula III and a hydroxy protective group reagent under the acid condition in a solvent;
c) hydrolyzing the compound of formula IV under the acid condition after the reaction of the compound of formula IV with a methylation reagent for obtaining 5(10),9(11)-diene-3,20-dione of formula V or 4(5),9(10)-dienen-3,20-dione or a mixture comprising both;
d) catalyzing the reaction of the compound of formula V and ethylene glycol with the p-toluenesulfonic acid and the trimethyl orthoformate or the triethyl orthoformate at the room temperature in dichloromethane for obtaining 3,3-(ethylene-dioxy)-17α-hydroxy-19-norpregna-5(10),9(11)-diene of formula VI;
e) epoxidizing the compound of formula VI by hydrogen peroxide for obtaining 3,3,20,20-bis(ethylendioxyl)-17α-hydroxyl-5,10-epoxy-19-norpregna-9(11)-ene of formula VII;
f) providing the addition reaction of the compound of formula VII and a 4-(N,N-diethyl amino) phenylmagnesium bromide Grignard reagent with cuprous chloride for obtaining 3,3,20,20-bis(ethylene-dioxy)-5α-17α-dihydroxy-11β-[4-(N,N-dimethylamino)-phenyl-]-19-norpregna-9(11)-ene of formula VIII;
g) hydrolyzing the compound of formula VIII under an acidic condition for obtaining 17α-hydroxy-11β-[4-(N,N-dimethylamino)-phenyl-]-19-norpregna-9(11)-diene-3,20-dione of formula IX; and
h) acetylating the compound of formula IX with acetic acid, perchloric acid and acetic anhydride at 0∼10°C in the dichloromethane for obtaining Ulipristal acetate of formula I.

2. The method for preparing Ulipristal acetate, as recited in claim 1, wherein the compound of formula III reacts with the hydroxyl protective group reagent for obtaining the compound of formula IV, the alkali is directly added for modifying a pH value to neutral or alkaline without separation, then the compound of formula IV reacts with methyl lithium or methyl Grignard reagent, the product was hydrolyzed in the solvent under the acid condition right after the reaction or after being processed for obtaining the compound in formula V.

3. A compound of formula IV, 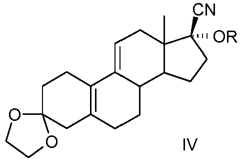 wherein R is defined in claim 1.

4. The compound as recited in claim 3, wherein said compound is selected from: 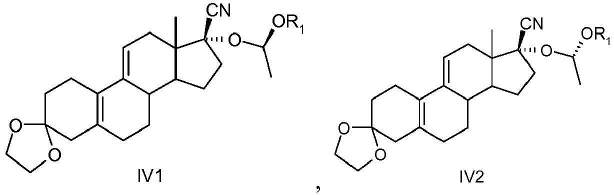 or 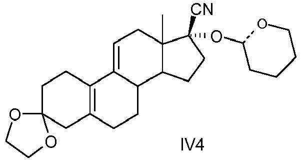 wherein R₁ and R₂ are defined in claim 1.

5. The compound as recited in claim 3 or 4, wherein said compound is selected from:

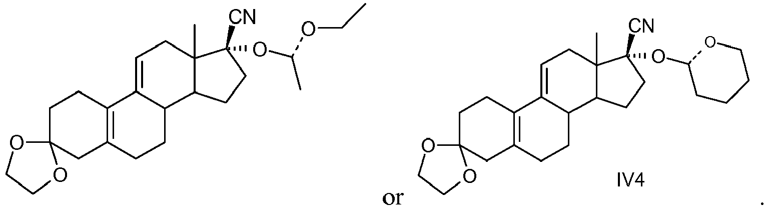

6. A method for preparing said compound of formula IV as recited in claim 3, comprising a step of providing a reaction of the compound of formula III and a hydroxyl protective group reagent for obtaining said compound of formula IV:

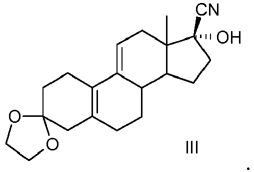

7. The method as recited in claim 6, wherein the hydroxyl protective group reagent is selected from vinyl ether or 2,3-dihydropyran, preferably, the vinyl ether is selected form ethyl vinyl ether, n-propyl vinyl ether, n-butyl vinyl ether, isobutyl vinyl ether and methyl vinyl ether.

8. A method for preparing said compound of formula V, 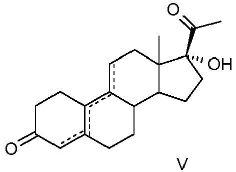 wherein dotted lines represents that locations of double bonds are at 5(10), 9(11) or 4(5), 9(10);
wherein the method comprises the steps of reacting the compound of formula IV with a methylation reagent and the hydrolysis of the product under acidic condition for obtaining the compound of formula V: 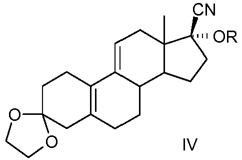 wherein R is defined in claim 1.

9. The method, as recited in claim 1 or 8, wherein the methylation reagent is methyl lithium or methyl Grignard reagent.

## Patentansprüche

1. Verfahren zur Herstellung von Ulipristalacetat, enthaltend die folgenden Reaktionsschritte: 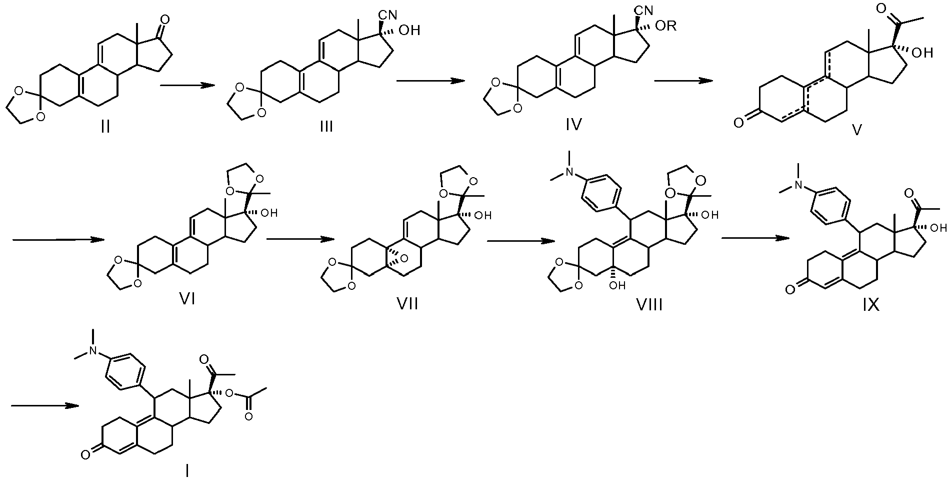 wobei R für eine Hydroxylschutzgruppe steht und aus -CH(CH₃)OR₁ oder 2-Tetrahydropyran gewählt ist, R₁ aus C1-C10 Alkyl- oder Arylrest gewählt ist; die gestrichelten Linien in Formel V die Stellung der Doppelbindungen bei 5(10),9(11), oder 4(5),9(10) zeigen,
wobei das Verfahren die folgenden Schritte enthält:
a) Verwendung von 3,3-(Ethylendioxyl)-19-norpregna-5(10),9(11)-dien-3,17-dion der Formel II als Rohmaterial und Verwendung von Alkoholen als Reaktionslösungsmittel, wobei 3,3-(Ethylendioxy)-17β-cyano-7α-hydroxyl-19-norpregna-5(10),9(11)-dien der Formel III durch Umsetzung des Ausgangsmaterials und eines Cyanierungsreagens unter schwach sauren Bedingungen bei einer Temperatur von -10°C bis Zimmertemperatur selektiv erhalten wird;
b) Herstellung der Verbindung der Formel IV durch Umsetzung der Verbindung der Formel III und eines Hydroxyschutzgruppen-Reagens unter sauren Bedingungen in einem Lösungsmittel;
c) Hydrolyse der Verbindung der Formel IV unter sauren Bedingungen nach Umsetzung der Verbindung der Formel IV mit einem Methylierungsreagens zum Erhalt von 5(10),9(11)-dien-3,20-dion der Formel V oder 4(5),9(10)-dienen-3,20-dion oder einer Mischung beider;
d) Katalysieren der Umsetzung der Verbindung der Formel V und von Ethylenglykol mit p-Toluolsulfonsäure und Trimethylorthoformiat oder Triethylorthoformiat bei Zimmertemperatur in Dichlormethan zum Erhalt von 3,3-(Ethylen-dioxy)-17α-hydroxy-19-norpregna-5(10),9(11)-dien der Formel VI;
e) Epoxidieren der Verbindung der Formel VI durch Wasserstoffperoxid zum Erhalt von 3,3,20,20-bis(Ethylendioxyl)-17α-hydroxyl-5,10-epoxy-19-norpregna-9(11)-en der Formel VII;
f) Durchführung der Additionsreaktion der Verbindung der Formel VII und eines 4-(N,N-Dimethylamino)phenylmagnesiumbromid-Crrignard-Reagens mit Kupfer(I)-chlorid zum Erhalt von 3,3,20,20-bis(Ethylen-dioxy)-5α-17α-dihydroxy-11β-[4-(N,N-dimethylamino)-phenyl-]-19-norpregna-9(11)-en der Formel VIII;
g) Hydrolyse der Verbindung der Formel VIII unter sauren Bedingungen zum Erhalt von 17α-Hydroxy-11β-[4-(N,N-dimethylamino)-phenyl-]-19-norpregna-9(11)-dien-3,20-dion der Formel IX; und
h) Acetylierung der Verbindung der Formel IX mit Essigsäure, Perchlorsäure und Essigsäureanhydrid bei 0∼10°C in Dichlormethan zum Erhalt von Ulipristalacetat der Formel I.

2. Verfahren zur Herstellung von Ulipristalacetat, wie im Anspruch 1 angegeben, wobei die Verbindung der Formel III mit dem Hydroxylschutzgruppen-Reagens zum Erhalt der Verbindung der Formel IV reagiert, das Alkali unmittelbar zugegeben ist, um den pH-Wert ohne Trennung auf neutral oder alkalisch einzustellen, dann die Verbindung der Formel IV mit Methyllithium oder Methyl-Grignard-Reagens reagiert, das Produkt in der Lösung unter sauren Bedingungen sofort nach der Umsetzung oder nach einer Aufarbeitung zum Erhalt der Verbindung der Formel V hydrolysiert wird.

3. Eine Verbindung der Formel IV, 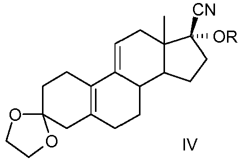 wobei R die im Anspruch 1 angegebenen Bedeutungen hat.

4. Die Verbindung nach Anspruch 3, wobei die Verbindung gewählt ist aus: 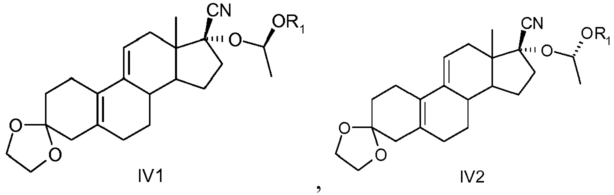 oder 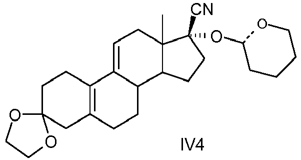 wobei R₁ und R₂ die im Anspruch 1 angegebenen Bedeutungen haben.

5. Die Verbindung nach Anspruch 3 oder 4, wobei die Verbindung gewählt ist aus:

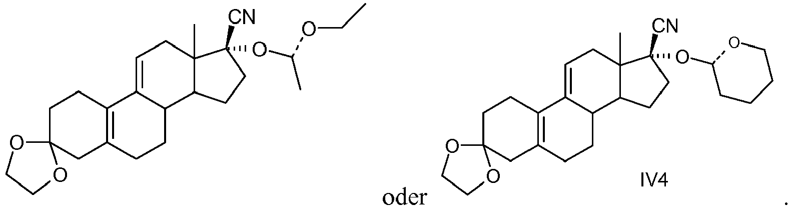

6. Verfahren zur Herstellung der Verbindung der Formel IV nach Anspruch 3, enthaltend den Schritt der Durchführung einer Umsetzung der Verbindung der Formel III und eines Hydroxylschutzgruppen-Reagens zum Erhalt der Verbindung der Formel IV:

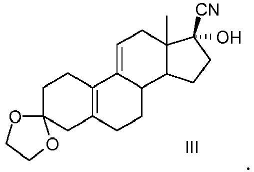

7. Das Verfahren nach Anspruch 6, wobei das Hydroxylschutzgruppen-Reagens aus Vinylether oder 2,3-Dihydropyran gewählt ist, der Vinylether bevorzugt aus Ethylvinylether, n-Propylvinylether, n-Butylvinylether, Isobutylvinylether und Methylvinylether gewählt ist.

8. Verfahren zur Herstellung der Verbindung der Formel V, 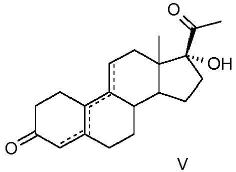 wobei die gestrichelten Linien die Stellung der Doppelbindungen bei 5(10), 9(11) oder 4(5), 9(10) zeigen;
wobei das Verfahren den Schritt der Umsetzung der Verbindung der Formel IV mit einem Methylierungsreagens und die Hydrolyse des Produkts unter sauren Bedingungen zum Erhalt der Verbindung der Formel V enthält: 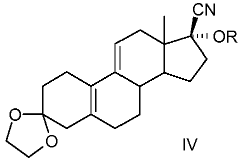 wobei R die im Anspruch 1 angegebene Bedeutung hat.

9. Das Verfahren nach Anspruch 1 oder 8, wobei das Methylierungsreagens Methyllithium oder Methyl-Grignard-Reagens ist.

## Revendications

1. Procédé pour la préparation de l'acétate d'Ulipristal, comprenant des étapes réactionnelles de: 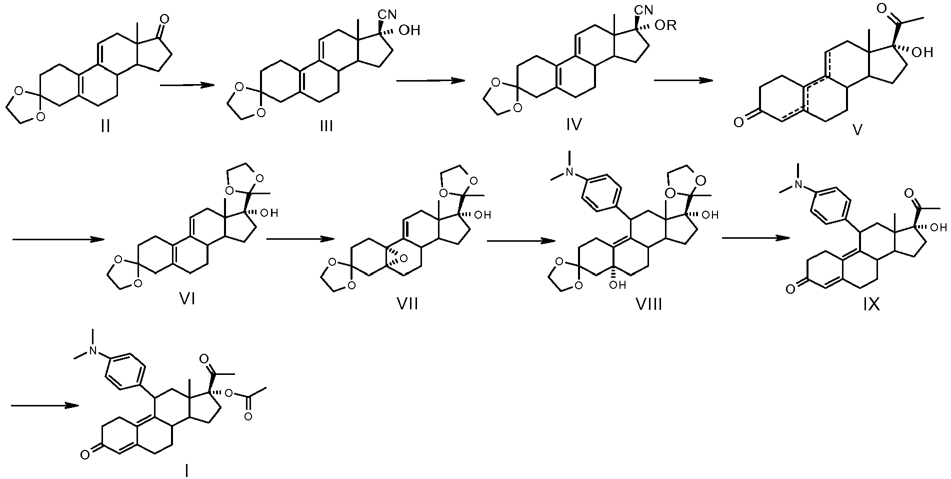 où R est un groupe protecteur de l'hydroxy, et est sélectionné parmi -CH(CH₃)OR₁, ou 2-tétrahydropyrane, R₁ est sélectionné respectivement parmi un radical alkyle en C1-C10 ou aryle ; les lignes pointillées dans la formule V représentent que les liaisons doubles sont situés à 5(10), 9(11), ou 4(5),9(10),
où le procédé comprend des étapes de :
a) utiliser 3,3-(éthylènedioxyl)-19-norprégna-5(10),9(11)-diène-3,17-dione de formule II comme matière première et utiliser des alcools comme solvant réactionnel, où 3,3-(éthylènedioxy)-17β-cyano-7α-hydroxyle-19-norprégna-5(10),9(11)-diène de formule III est obtenu sélectivement par une réaction de la matière première et un réactif de cyanation dans des conditions d'acidité faible à une température comprise entre -10°C et la température ambiante ;
b) obtenir le composé de formule IV en faisant réagir le composé de formule III et un réactif de groupe protecteur de l'hydroxy dans les conditions acides dans un solvant ;
c) hydrolyser le composé de formule IV dans les conditions acides après la réaction du composé de formule IV avec un réactif de méthylation pour obtenir 5(10),9(11)-diène-3,20-dione de formule V ou 4(5),9(10)-diène-3,20-dione ou un mélange comprenant les deux ;
d) catalyser la réaction du composé de formule V et l'éthylène glycol avec l'acide p-toluènesulfonique et l'orthoformiate de triméthyle ou l'orthoformiate de triéthyle à température ambiante dans du dichlorométhane pour obtenir le 3,3-(éthylène-dioxy)-17α-hydroxy-19-norprégna-5(10),9(11)-diène de formule VI ;
e) l'époxydation du composé de formule VI par du peroxyde d'hydrogène pour obtenir le 3,3,20,20-bis(éthylendioxyl)-17α-hydroxyl-5,10-époxy-19-norprégna-9(11)-ène de formule VII;
f) assurer la réaction d'addition du composé de formule VII et un réactif de Grignard bromure de 4-(N,N-diméthylamino)phénylmagnésium avec du chlorure cuivreux pour obtenir le 3,3,20,20-bis(éthylène-dioxy)-5α-17α-dihydroxy-11β-[4-(N,N-diméthylamino)-phényl-]-19-norprégna-9(11)-ène de formule VIII;
g) hydrolyser le composé de formule VIII dans des conditions acides pour obtenir le 17α-hydroxy-11β-[4-(N,N-diméthylamino)-phényl-]-19-norprégna-9(11)-diène-3,20-dione de formule IX; et
h) acétyler le composé de formule IX avec l'acide acétique, l'acide perchlorique et l'anhydride acétique à 0∼10°C dans le dichlorométhane pour obtenir l'acétate d'Ulipristal de formule I.

2. Le procédé pour la préparation de l'acétate d'Ulipristal, selon la revendication 1, où le composé de formule III réagit avec le réactif de groupe protecteur de l'hydroxy pour obtenir le composé de formule IV, l'alcali est directement ajouté pour modifier le pH à neutre ou alcalin sans séparation, ensuite le composé de formule IV réagit avec le méthyl lithium ou le réactif de Grignard méthyle, le produit a été hydrolysé dans le solvant dans les conditions acide immédiatement après la réaction ou après traitement pour obtenir le composé de formule V.

3. Un composé de formule IV, 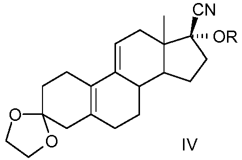 où R est tel que défini dans la revendication 1.

4. Le composé selon la revendication 3, où ledit composé est sélectionné parmi : 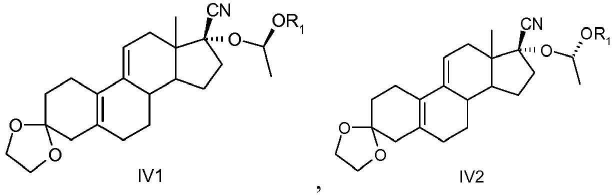 ou 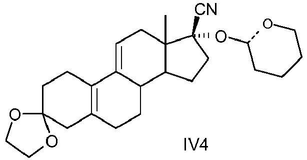 où R₁ et R₂ sont tels que définis dans la revendication 1.

5. Le composé selon les revendications 3 ou 4, où ledit composé est sélectionné parmi :

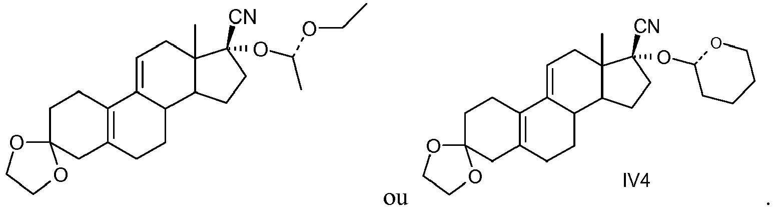

6. Procédé pour la préparation dudit composé de formule IV selon la revendication 3, comprenant une étape d'assurer une réaction du composé de formule III et d'un réactif de groupe protecteur de l'hydroxyle pour obtenir ledit composé de formule IV:

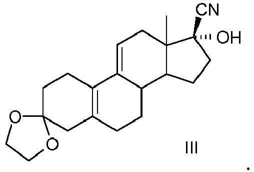

7. Le procédé selon la revendication 6, où le réactif de groupe protecteur de l'hydroxyle est sélectionné parmi un éther vinylique ou 2,3-dihydropyrane, l'éther vinylique est sélectionné parmi l'éther vinylique d'éthyle, l'éther vinylique de n-propyle, l'éther vinylique de n-butyle, l'éther vinylique d'isobutyle et l'éther vinylique de méthyle.

8. Procédé pour la préparation dudit composé de formule V, 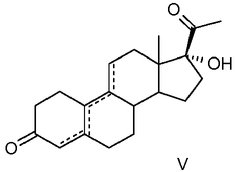 où les lignes pointillées représentent que les liaisons doubles sont situés à 5(10), 9(11), ou 4(5) ,9(10),
où le procédé comprend les étapes de faire réagir le composé de formule IV avec un réactif de méthylation et l'hydrolyse du produit dans les conditions acides pour obtenir le composé de formule V: 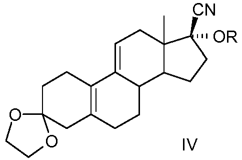 où R est tel que défini dans la revendication 1.

9. Le procédé selon les revendications 1 ou 8, où le réactif de méthylation est le méthyle lithium ou un réactif de Grignard méthyle.
